# EUROPEAN PATENT SPECIFICATION

(11) **EP 1 814 507 B1**
(45) Date of publication and mention of the grant of the patent: **03.11.2010**
(21) Application number: 05846805.9
(22) Date of filing: 18.11.2005
(51) Int. Cl.: A61K 6/083, A61K 6/09

(54) **DENTAL COMPOSITE RESTORATIVE MATERIAL**
DENTALER REPARATURVERBUNDSTOFF
MATIERE COMPOSITE DE RESTAURATION DENTAIRE

(30) Priority: 23.11.2004 US 630495 P
(43) Date of publication of application: 08.08.2007
(73) Proprietor: DENTSPLY International Inc., York, PA 17405-0872 (US)
(72) Inventor: HARE, Robert, V., Georgetown, DE 19947 (US)
(74) Representative: Hartz, Nikolai
(86) International application number: PCT/US2005/041865
(87) International publication number: WO 2006/057908

(56) References cited:
- EP-A- 0 166 009
- US-A- 5 179 186

## Description

### Technical Field

The present invention according to claim 1, is dental restorative material for use for use in for example, class 1 & 2 posterior cavity restorations. The inventive material includes a modified BisGma resin. The invention can be used in a method of restoring a damaged tooth that includes the application of bonding agent, warming the material so that it can be extruded, and placing the material into the tooth cavity. Once the material is , placed in the tooth it can be packed with conventional composite instruments, shaped, carved to match tooth anatomy, then cured by visible light energy. It is intended that this material will be able to be placed into the tooth cavity in one or two increments. Due to this materials low shrinkage value, it can be placed into the tooth cavity without buildup of significant stress forces. In some cases, because of the desire for aesthetic restorations, a body shade can be placed in cured and then covered with an incisial shade and cured. These techniques are intended to be quicker to complete than traditional layering composite techniques and more like the placement of amalgam.

### Background of the Invention

Dental composites, which often contain a mixture of a polymerizable resin and a filler, have been developing since the early 1970's, when the first materials of this class were introduced. Until this time, fillings had been based on silver-mercury amalgams, mixtures of acid leachable glass with phosphoric acid (known as "silicate cements"), or unfilled polymerizable resins, and each class of material has certain strengths and weaknesses. For instance, amalgams are generally considered to be cheap and easy to use, and to have a long lifetime due to their strength and high resistance to wear. Disadvantages of amalgam are toxicity of the mercury and the black colour of the filling. Silicate fillings were approximately tooth coloured and released fluoride into the tooth to help prevent a recurrence of decay. However they tended to dissolve quickly and were weak, and are barely used nowadays. Unfilled resins brought advantages of toughness, convenience, and aesthetics, but were still weak, limiting their use to areas of low stress. These unfilled resins also have a high volume shrinkage, often as high as 5%. This leads to formation of gaps between the filling and the tooth, and subsequent recurring decay of the tooth around and underneath the restoration. The introduction of composite materials brought improvements in surface hardness, higher physical strengths, good aesthetics, lower shrinkage, and also higher resistance to wear. However the wear rate of these composite materials was often still higher than of amalgam, and their shrinkage of around 2 to 3 volume percent still leads to gap formation and recurrent caries. It is an aim of many researchers in the dental area to develop composite materials with higher strength, reduced shrinkage and higher resistance to wear, which may be used in place of amalgam.

Certain practical considerations apply to the formulation and application of dental restorative, such as resin-based posterior dental restoratives. Accordingly, it is greatly preferred that the restorative composition be effectively homogeneous such that air bubbles or structural discontinuities are substantially avoided from introduction into the tooth structure. Additionally, it is preferred that such materials be "packable" or "condensable" and be capable of deforming a matrix band during the course of tooth filling. Such materials should also be capable of withstanding the physical stresses extant in the posterior region of the mouth and not crumble, fracture or erode under such conditions.

It has long been known to employ metallic amalgams in the restorations of posterior teeth. Such amalgam materials have been shown to have good resistance to the physical stresses experienced by posterior teeth and to posses small coefficients of thermal expansion. Such amalgams have also been demonstrated to have good "packability" and to demonstrate other properties necessary of the posterior restorative. Such materials however, suffer from uncertainty as to the biological effect of the introduction of mercury and other materials in the oral cavity over long periods of time.

Those skilled in the art of dental restoration will appreciate that certain posterior restorations, such as Class 2 restorations, require the employment of a matrix for proper application. This is to ensure that the replacement of the natural tooth structure is replaced and restored in close contact with the adjacent tooth. Thus, it will be appreciated that the use of a matrix band to surround the tooth to be repaired is generally necessary. Such bands are needed when the tooth to be repaired must be excavated in such a fashion that the resulting cavity preparation communicates from the top surface to one or more of the side surfaces of the tooth. In such a case, the matrix band is placed around the tooth and held tightly in place while a restorative material such as amalgam is put into place. A measure of a material's packability and values of the measured packability for conventional dental amalgams is described for example in U.S. Pat. No. 4,226,622. Heretofore, conventionally and commercially available composite materials, while durable, have suffered from low packability values. This results in less than effective distention of the matrix band, often resulting in "rebound" or recovery of the band's original shape and ultimately to less than ideal contacts.

EP-A 0 166 009 discloses a photopolymerizable composition comprising a reaction product of a polyisocyanate and a diglycidyl (meth)acrylate of a bisphenol.

The present invention is different than traditional composite materials, because traditional composite materials that are stiff at room temperature become soft and sticky as they are manipulated and reach oral temperature. As traditional materials become softer due to manipulation and oral temperature, they also have increased flow characteristics that makes it difficult for the clinician to shape occlusal detail because the material will slump due to its increased flow at oral temperature. The present inventive material can be bulk placed, packed, carved, and cured with minimal effort in a very short period of time.

### Summary of the Invention

The present invention provides a dental restorative material according to claim 1. According to one embodiment of the invention, there is provided a composite that contains mixtures of the following: modified BisGma resin, triacrylate resin known as Sartomer SR 444, radiopaque fluoride glass, photoinitiators, inhibitors, flublau, Uvinul M-40, and pigments.

The present invention also provides a method of making a dental restorative according to claim 5. According to an inventive method, the above composition is prepared by mixing in a high torque double planetary mixer that is equipped with temperature control and vacuum capability. The mixer temperature is set to about 50 to 60°C so that the composite remains soft and pliable while it is being compounded. Vacuum is applied to the paste in the last step of the compounded procedure to remove porosity from the paste.

### Preferred Embodiments for Carrying Out the Invention

The present invention provides a dental restorative material, useful for example, in Class 2 and 3 posterior restoration, that includes a mixture of preferably, modified BisGma resin, triacrylate resin known as Sartomer SR 444, radiopaque fluoride glass, photoinitiators, inhibitors, flublau, Uvinul M-40, and pigments.

The modified BisGma resin is the reaction product of BisGma and HMDI. The hydroxyl groups on the BisGma are reacted with the isocyanate, so that 40 to 60% of all the available hydroxyls are reacted. The reaction is carried out in a double planetary mixer at 50°C. This reaction can be conducted neat or with the addition of the SR 444. The fact that the modified BisGma can be made neat goes against the teachings of other inventors. They claimed that these resins can only be made in solvents or highly diluted with other monomers in order to reduce the viscosity to a point where they can be mixed in a reactor. By using a high torque double planetary mixer we have been able to produce resins of extremely high viscosities, which have between 10 and 90% of the available hydroxyl reacted. The advantage to producing resin in this manner is demonstrated by the unique handling characteristics and low shrinkage of composites made with these resins. The viscosity and or molecular weight effect the handling characteristics of composites made with these resins. The molecular weight of the resin controls the thermal transition point at which the composite goes from solid to an extrudable material. The higher the molecular weight, the higher the temperature, that is required to cause the material to be extrudable. The goal of this invention is to have a material that is extrudable between 50 and 60°C and becomes packable when it reaches oral temperature of 35 to 37°C. This change of state also makes the material less sticky it easier to handle.

Modified BisGma resin composition:

| Ingredient | Preferred Percent by weight | Percent range |
|---|---|---|
| BisGma | 85.645 | 80 to 90% |
| HMDI Hexamethylene diisocyanate | 14.05 | 10 to 20% |
| T9® catalyst | 0.005 | 0.001 to .05% |
| Butylated hydroxy toluene (BHT) | 0.15 | 0 to 0.3% |
| MEHQ | 0.15 | 0 to 0.3% |

The ingredient SR 444 -- pentaerythritol triacrylate, has been added to increase strength of the final composite. As with other composites, the additions of other methyacrylate monomers improve strength. But as the down side also increase percent shrinkage. By using SR 444, which is a triacrylate instead of a diacrylate, a much lower percentage can be used in the final composition to improve strength and still maintain a low percent shrinkage.

The filler used in this composition is a blend of different particle sizes of a fluoridated barium boron aluminum silicate glass known as Ferro EG 9726 glass. This glass is milled to three different mean particle sizes (1, 6, 17 µm (Micron)) and each is silanated. These three glasses are used together in a filler blend that composes 78 to 82% of the total filler make up of the final composite. These filler blend leads to low wear characteristics, optical translucency, and good handling.

The photoinitiators CQ and EDAB are used so that this one component composite can be visibly light cured.

The inhibitors, BHT and MEHQ, are used in the composition to prevent premature gelation of the resin and composite or in processing and storage of the final composition.

The pigments used in this system are titanium and iron oxides. These pigments can be used, where shading is desired.

The formulation will also include a fluorescent agent and a UV stabilizer.

| Ingredient | Percent by weight | Percent range |
|---|---|---|
| Modified BisGma resin | 18 | 15-20 |
| SR 444 (pentaerythritol triacrylate) | 2 | 0 - 5 |
| Camphorquione (CQ) | 0.2 | 0.05 - 0.5 |
| Ethyl DiaminoBenzoate (EDAB) | 0.8 | 0.08-2.0 |
| Flublau (fluorescent agent) | 0.04 | 0-1.0 |
| Uvinul M40 | 0.15 | 0-1.0 |
| Silanated Ferro EG 9726 VSD (17 µm (mn.) mean) | 9.85 | 0-20 |
| Silanated Ferro EG 9726 (6 µm (mn.) mean) | 59.06 | 30 - 80 |
| Silanated Ferro EG 9726 ultrafine (1 µm (mn.) mean) | 9.85 | 0-20 |
| Iron oxide pigments | 0.05 | 0 - 0.5 |

The above composition is prepared by mixing in a high torque double planetary mixer that is equipped with temperature control and vacuum capability. The mixer temperature is set that 50 to 60°C so that the composite remains soft and pliable while it is being compounded. Vacuum is applied to the paste in the last step of the compounded procedure to remove porosity from the paste.

According to one aspect of the invention, this composition is different from traditional methacrylate based dental composites in that it is designed to have low wear, low shrinkage, and unique handling characteristics. The present invention material has handling characteristics very similar to traditional amalgam in that the material goes from a soft, easy to extrude state at 60°C and becomes stiff and packable when it reaches oral temperature. As a matter of fact, the material is a solid at room temperature, and can only be used when heated to 40 to 60°C. At this temperature range, the material becomes extrudable and can be placed into a tooth preparation using a bulk filling technique. As the material becomes cooler, reaching oral temperature of about 37°C, its viscosity increases and the material becomes packable. This characteristic is very different than traditional composite materials, because traditional composite materials that are stiff at room temperature become soft and sticky as they are manipulated and reach oral temperature. As traditional materials become softer due to manipulation and oral temperature, they also have increased flow characteristics that makes it difficult for the clinician to shape occlusal detail because the material will slump due to its increased flow at oral temperature. The Present inventive material can be bulk placed, packed, carved, and cured with minimal effort in a very short period of time.

An advantage to producing composite of the present invention is demonstrated by the unique handling characteristics and low shrinkage of composites made with these resins. The viscosity and/or molecular weight effect the handling characteristics of composites made with these resins. The molecular weight of the resin controls the thermal transition point (See the attached Chart 1 that illustrates the effect of the number of hydroxyl's react on the thermal transition point) at which the composite goes from solid to an extrudable material. The higher the molecular weight, the higher the temperature, that is required to cause the material to be extrudable. The goal of this invention is to have a material that is extrudable between 50 and 60°C and becomes packable when it reaches oral temperature of 35 to 37°C. This change of state also makes the material less sticky and easier to handle.

The present inventive composite may benefit from an energy assisted delivery system or in other words, a way to heat or impart energy to the material so that it can be extruded from a storage device into the tooth. This is due to the fact that the material is nearly solid at 23°C and cannot be extruded from the package without applying energy to the material first, such as by warming it. For example, this may be accomplished by using a microwave oven to heat the material in the package or compule. It was found that 25 seconds on high, in the microwave, allowed the material to remain flowable for 1.5 minutes. In another embodiment, a device known as an Adent compule warmer was utilized. The Adent compule warmer was set to 60°C. Other approaches include for example, heated compule guns, ultrasonic delivery, and the like.

The present inventive has handling characteristics that separate it from other known composites. The material has low shrinkage, low wear and good physical properties. The present inventive material can be bulk placed, packed, carved, and cured in the matter of the few minutes.

The attached table labeled 46 series DOE results contains typical properties of the present composites. In this table the resins that were 50, 55, and 60% reacted were used in combination with SR 444 at 0, 2, 4, 6, and 8% of the total composite makeup. The responses or physical properties tested were abrasion loss (shown as a profilometer volume loss), percent shrinkage, compressive strength, and push test. The abrasion test used is known as prophy abrasion. In this as a sample of the test composite is repeatedly exposed to abrasion using coarse prophy paste and a webbed prophy cup. After 16 cycles of abrasion the volume loss was measured using a profilometer. Percent shrinkage was measured using the Archimedes method based on density of the uncured material and density of the cured material. From the two density values the percent of volume metric shrinkage was calculated. Samples were also prepared for measuring compressive strength. The samples were 4 x 6mm cylinders that were fully cured using visible light. The samples were then stored in water and 37°C for 24 hours before testing on and Instron. The push test was developed using an AR -1000 advanced rheometer. In this test the material temperature was controlled to 37°C and the force in Newtons was recorded that resulted from pushing the material 1 1/2 millimeters. This test was done to stimulate the packing force required at oral temperature.

The results of these mostly testing show that when the percent of SR 444 was increased, the strength increase, but the percent shrinkage also increased. Even so, the percent shrinkage of the present composite design of experiments was generally lower than the competitive materials.

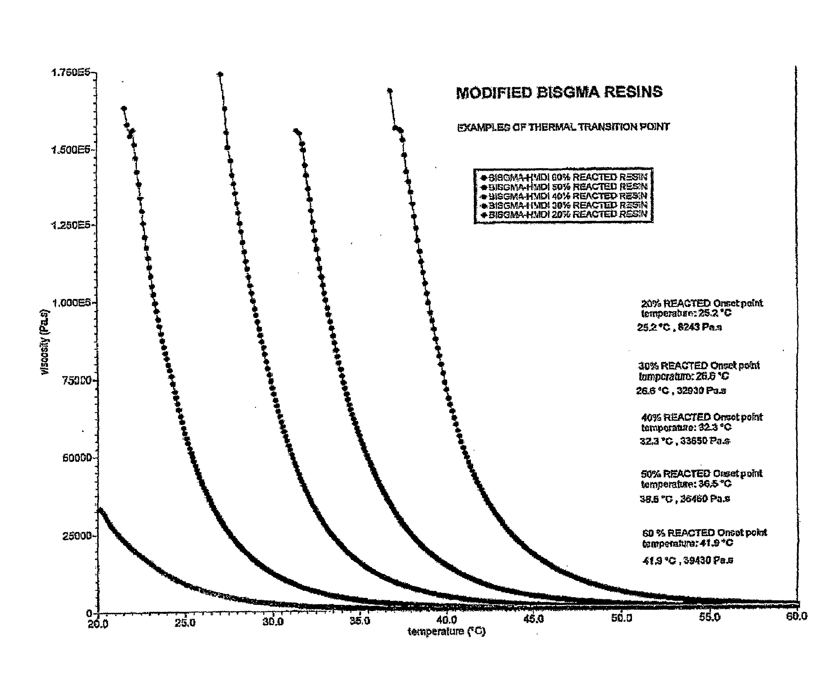

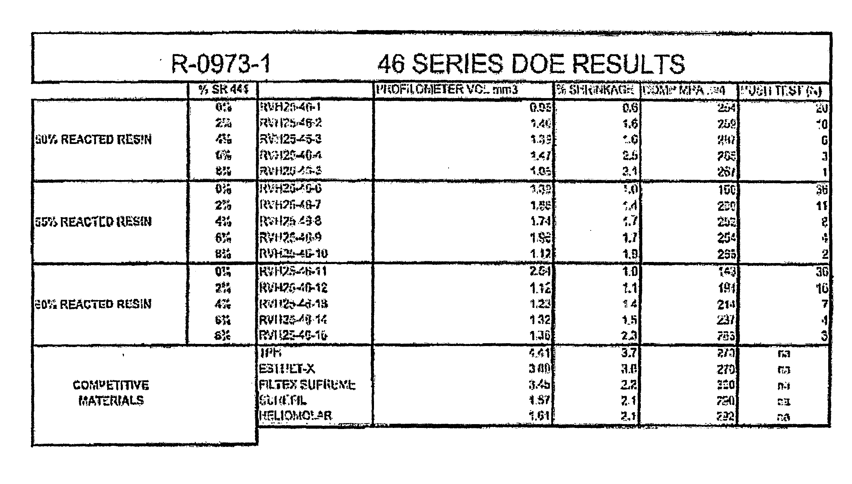

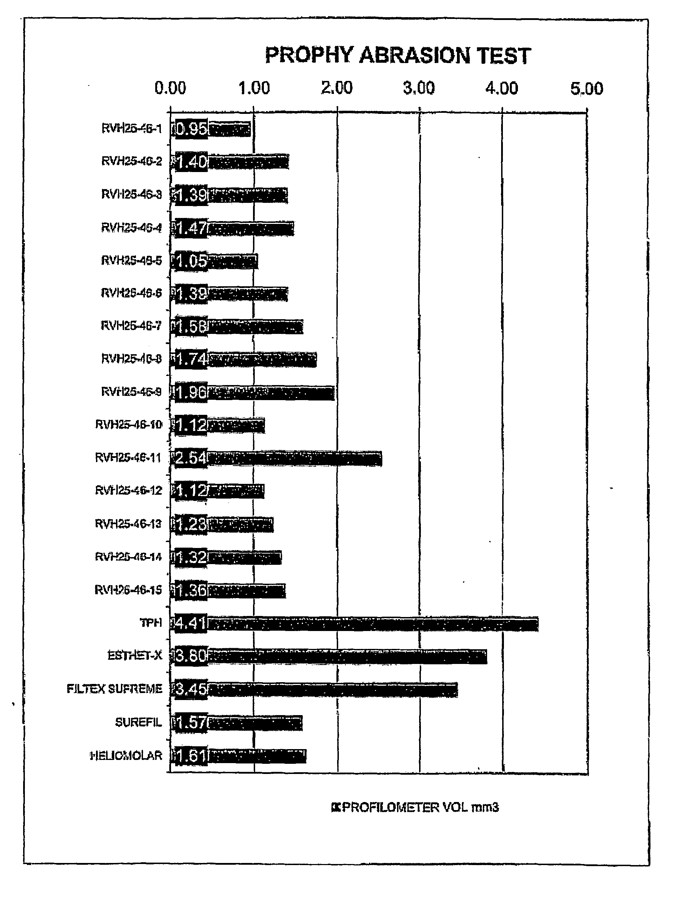

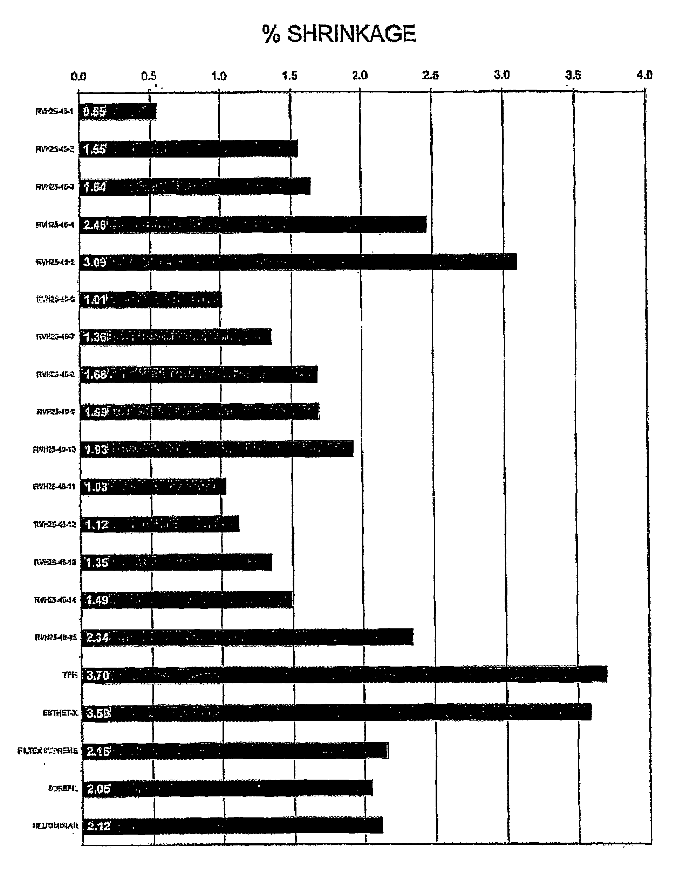

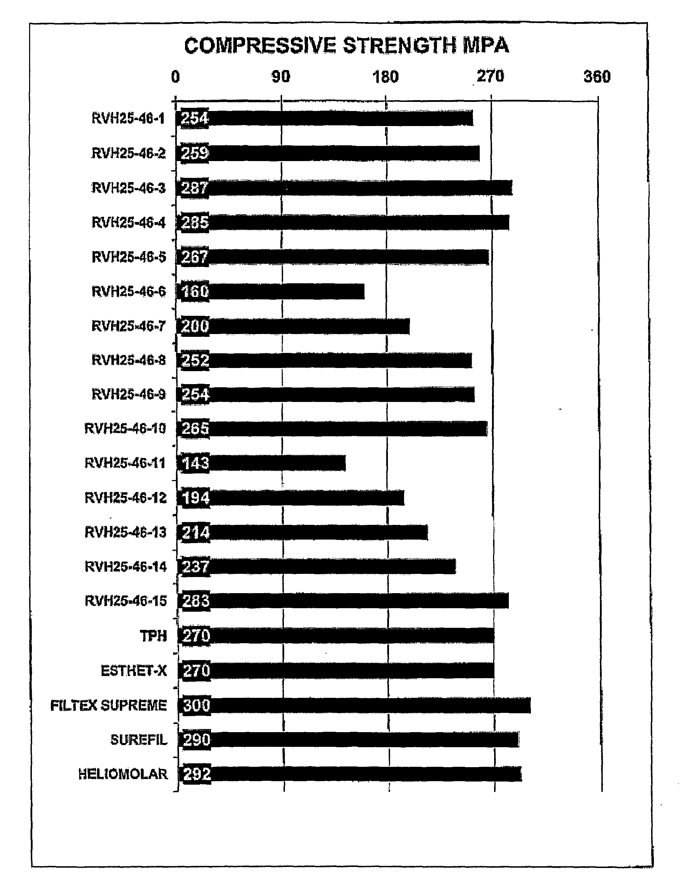

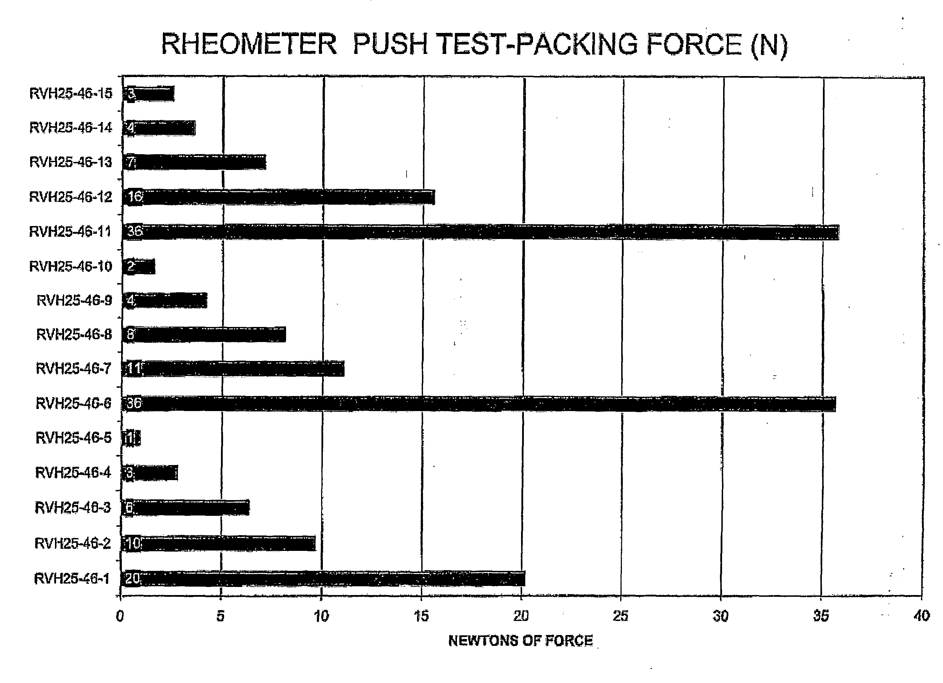

## Claims

1. A dental restorative material comprising a modified BisGMA resin being the reaction product of BisGMA and hexamethylene diisocyanate, which is obtainable by reacting 40 to 60% of all the available hydroxyl groups on the BisGMA with the isocyanate in a mixture comprising by weight
| | |
|---|---|
| 80 to 90 % | BisGMA |
| 10 to 20 % | hexamethylene diisocyanate |
| 0.001 to 0.05 % | T9^{®} catalyst |
| 0 to 0.3% | BHT |
| 0 to 0.3 % | MEHQ |
said dental restorative material being extrudable above 40 °C and being a solid at room temperature.

2. A dental material as in claim 1, further comprising a photoinitiator.

3. A dental material as in claim 1, further comprising a filler material.

4. A dental material as in claim 1, further comprising pentaerythritol triacrylate.

5. A method of making a dental restorative as defined by any one of claims 1 to 3, comprising the step of neat reacting BisGMA and hexamethylene diisocyanate under high torque.

6. A method according to claim 5, wherein the hydroxyl groups on the BisGMA are reacted with the isocyanate, so that 40 to 60% of all the available hydroxyls are reacted.

7. A method according to claim 5 or 6, wherein a high torque double planetary mixer equipped with temperature control and vacuum capability is used.

8. Use of a reaction product of BisGMA and hexamethylene diisocyanate as defined by any one of claims 1 to 4, for the manufacture of a dental restorative material for restoring a tooth by heating the restorative material and extruding it onto a prepared tooth site.

## Patentansprüche

1. Dentales Wiederherstellungsmaterial, umfassend ein modifiziertes Bis-GMA-Harz, das ein Reaktionsprodukt aus BisGMA und Hexamethylendiisocyanat ist, das erhältlich ist durch Umsetzung von 40 bis 60 % aller zur Verfügung stehenden Hydroxylgruppen des BisGMA mit dem Isocyanat in einem Gemisch, umfassend nach Gewicht
| | |
|---|---|
| 80 bis 90% | BisGMA |
| 10 bis 20% | Hexamethylendiisocyanat |
| 0,001 bis 0,05% | T9® Katalysator |
| 0 bis 0,3% | BHT |
| 0 bis 0,3% | MEHQ |
wobei das dentale Wiederherstellungsmaterial oberhalb von 40°C extrudierbar ist und bei Raumtemperatur fest ist.

2. Das Dentalmaterial nach Anspruch 1, das ferner einen Photoinitiator umfasst.

3. Das Dentalmaterial nach Anspruch 1, das ferner einen Füllstoff enthält.

4. Das Dentalmaterial nach Anspruch 1, das ferner Pentaerythritoltriacrylat umfasst.

5. Verfahren zur Herstellung eines dentalen Wiederherstellungsmaterials, wie in einem der Ansprüche 1 bis 3 definiert, das die Stufe einer reinen Umsetzung von BisGMA und Hexamethylendiisocyanat unter hohem Drehmoment umfasst.

6. Verfahren nach Anspruch 5, wobei die Hydroxylgruppen auf dem BisGMA umgesetzt werden mit dem Isocyanat, so dass 40 bis 60% aller zur Verfügung stehenden Hydroxylgruppen umgesetzt werden.

7. Verfahren nach Anspruch 5 oder 6, wobei ein Doppelplanetenmischer mit hohem Drehmoment eingesetzt wird, der eine Temperaturkontrolle und eine Fähigkeit zum Unterdruck aufweist.

8. Verwendung eines Reaktionsprodukts aus BisGMA und Hexamethylendiisocyanat, wie es in einem der Ansprüche 1 bis 4 definiert wird, zur Herstellung eines dentalen Wiederherstellungsmaterials zur Wiederherstellung eines Zahns durch Erhitzen des Wiederherstellungsmaterials und dessen Extrudierung auf eine vorbereitete Zahnstelle.

## Revendications

1. Matériau de reconstitution dentaire, comprenant une résine de BisGMA modifiée qui consiste en le produit de réaction de BisGMA et d'hexaméthylène-diisocyanate, qui peut être obtenu en faisant réagir 40 à 60 % de tous les groupes hydroxyle disponibles sur le BisGMA avec l'isocyanate dans un mélange comprenant, en poids
| | |
|---|---|
| 80 à 90 % | de Bis GMA |
| 10 à 20 % | d'hexaméthylène-diisocyanate, |
| 0,001 à 0,05 % | de catalyseur T9^{®} |
| 0 à 0,3 % | de BHT |
| 0 à 0,3 % | de MEHQ |
ledit matériau de reconstitution dentaire étant extrudable à une température supérieure à 40 °C et étant une matière solide à température ambiante.

2. Matériau dentaire suivant la revendication 1, comprenant en outre un photo-initiateur.

3. Matériau dentaire suivant la revendication 1, comprenant en outre une matière servant de charge.

4. Matériau dentaire suivant la revendication 1, comprenant en outre du triacrylate de pentaérythritol.

5. Procédé pour la préparation d'un matériau de reconstitution dentaire, tel que défini par l'une quelconque des revendications 1 à 3, comprenant l'étape consistant à faire réagir à l'état pur du BisGMA et de l'hexaméthylène-diisocyanate dans des conditions de haut moment de torsion.

6. Procédé suivant la revendication 5, dans lequel les groupes hydroxyle sur le BisGMA sont amenés à réagir avec l'isocyanate, de telle sorte que 40 à 60 % de tous les groupes hydroxyle disponibles réagissent.

7. Procédé suivant la revendication 5 ou 6, dans lequel un mélangeur planétaire double à haut moment de torsion équipé de moyens de régulation de température et de mise sous vide est utilisé.

8. Utilisation d'un produit de réaction de BisGMA et d'hexaméthylène-diisocyanate tel que défini par l'une quelconque des revendications 1 à 4, pour la production d'un matériau de reconstitution dentaire destiné à la reconstitution d'une dent par chauffage du matériau de reconstitution et extrusion de celui-ci sur un site dentaire préparé.
